(19) Europäisches Patentamt
European Patent Office
Office européen des brevets

(11) Publication number: **0 464 781 B1**

# (12) EUROPEAN PATENT SPECIFICATION

(45) Date of publication of patent specification: **02.11.94**

(51) Int. Cl.$^5$: **C08G 18/70**, C08G 18/64, C08G 18/71, C08G 18/28, C08L 97/02, C07C 263/18

(21) Application number: **91110963.5**

(22) Date of filing: **02.07.91**

## (54) Process for the preparation of aqueous polyisocyanate emulsions and emulsions obtained thereby.

(30) Priority: **03.07.90 IT 2084690**

(43) Date of publication of application:
**08.01.92 Bulletin 92/02**

(45) Publication of the grant of the patent:
**02.11.94 Bulletin 94/44**

(84) Designated Contracting States:
**AT BE CH DE DK FR GB GR LI NL SE**

(56) References cited:
**EP-A- 0 013 112**
**EP-A- 0 335 342**
**US-A- 3 899 387**

(73) Proprietor: **MINISTERO DELL' UNIVERSITA' E DELLA RICERCA SCIENTIFICA E TECNOLOGICA**
**76, Lungotevere Thaon de Revel**
**I-00196 Roma (IT)**

(72) Inventor: **Lepori, Agostino**
**22, Via Legnano**
**I-21054 Fagnano Olona Varese (IT)**
Inventor: **Camaioni, Domenico**
**24, Via Rovereto**
**I-21052 Busto Arsizio Varese (IT)**

(74) Representative: **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

**EP 0 464 781 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of aqueous polyisocyanate emulsions, to the emulsions thus obtained and the use thereof as binding agents for cellulosic materials.

The term "polyisocyanate" as used in the present description and in the claims denotes organic compounds of low, medium and/or high molecular weight which contain at least two -NCO groups.

The low molecular weight polyisocyanates are diisocyanates of general formula (I):

OCN-R-NCO (I)

wherein R represents an alkyl, cycloalkyl, aryl or alkylaryl radical having from 1 to 25, particularly 6 to 20 carbon atoms such as meta- and/or para-phenylene diisocyanate, 2,4-toluene diisocyanate, either alone or mixed with 2,6-toluene diisocyanate, 4,4'-diphenyl-methane diisocyanate, hexamethylene diisocyanate, 4,4'-dicyclohexyl-methane-diisocyanate, 1-isocyanato-3-isocyanato-methyl-3,5,5-trimethylcyclohexane (or isophorone diisocyanate), 2,2,4-trimethylhexamethylene diisocyanate, optionally in admixture with the 2,4,4-trimethylhexamethylene diisocyanate isomer, etc.

The medium and high molecular weight polyisocyanates are those of different degrees of condensation which can be obtained from the phosgenation of aniline-formaldehyde condensates. These products are composed of mixtures of polymethylene polyphenylisocyanates of general formula (II):

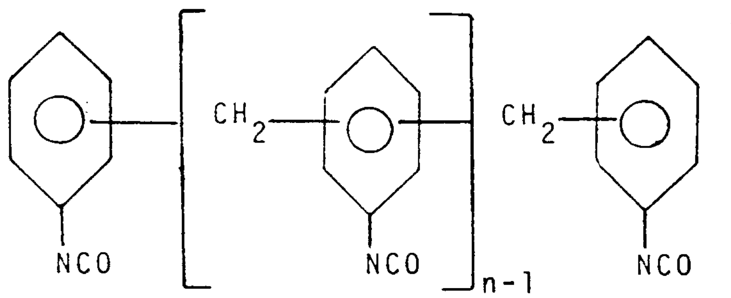

(II)

wherein n represents an integer higher than or equal to 1.

Particularly preferred polyisocyanates are the mixtures of polymethylene polyphenylisocyanates having an average functionality of about 2.6 to about 2.8; such products are marketed under various trade names such as Tedimon(R) 31(Montedipe), PaPi(R) (Upjohn) and Mondur(R) MR (Mobay).

The use of both aliphatic and aromatic diisocyanates and polyisocyanates, particularly in the form of aqueous emulsions, as binding agents for preparing wood composites is known, e.g., from EP-A-13112.

Said emulsions can be sprayed onto wood chips which form a mat which, thereafter, is subjected to elevated temperatures and pressures to prepare a particle board.

In order to be able to use aqueous emulsions of polyisocyanates as binding agents in wood composites it is very important that said emulsions do not exhibit too limited a chemico-physical stability, so as to be suitable for the adjustment to different drawbacks which may occur during industrial processes and which are not always foreseeable.

The term "chemico-physical stability" relates to both the retainment of the physical properties of the emulsions over time and to a substantial chemical stability over time of the isocyanate groups as regards their reaction with water.

In EP-A-335342 aqueous polyisocyanate emulsions, stabilized by particular cellulose ethers having a substitution degree higher than 0.5 and providing a viscosity of the 2% aqueous solution between 10 and 45000 mPa•s, are described.

These compositions show a satisfactory chemical and physical stability but they can be obtained only by mixing water, polyisocyanate and cellulose ethers at very high agitation speeds, for instance, by using turbostirrers.

However, this makes the production of emulsions more expensive when the operation is carried out on an industrial scale, particularly because of the large volumes of raw materials used.

It has now been found that stable aqueous polyisocyanate emulsions can be obtained by adding to the cellulose ethers of the prior art a surface-active agent which is derived from phenylisocyanate.

Therefore the present invention provides a process for the preparation of aqueous polyisocyanate emulsions which comprises the mixing in water, under mild agitation, of

(i) at least one cellulose ether in an amount of from 0.1 to 10% by weight, based on the total weight of water and cellulose ether;

(ii) at least one surface-active agent derived from phenylisocyanate and having the general formula (III):

$$X-(C_mH_{2m}O)_n-CONHY \qquad (III)$$

wherein m is an integer of from 1 to 4;
n is an integer of from 5 to 40;
X represents an -OH, a $C_1$-$C_4$ alkoxy or a -CONHY group; and
Y represents a phenylisocyanate residue; in an amount of from 0.5 to 2% by weight of the final emulsion; and

(iii) at least one polyisocyanate of general formulae (I) and (II) as defined above.

Preferred examples of surface-active agents of general formula (III) are those obtained from the reaction between phenylisocyanate and polyoxyethylene glycols having an average molecular weight of from 200 to 1500, polyoxypropylene-ethylene glycols having an average molecular weight of from 200 to 1500, oxyethylated butanol having an average molecular weight of from 400 to 1000, oxyethylated methanol having an average molecular weight of from 500 to 2000, butanol oxyethylene-oxypropylene alcohol having an average molecular weight of from 500 to 1000, methanol oxyethylene-oxypropylene alcohol having an average molecular weight of from 500 to 1000.

Preferably, the cellulose ether is dissolved in water, at room temperature, in an amount of from 0.5 to 5% by weight, based on the total weight of water and cellulose ether. Mechanical stirring at 1000 to 2000 rpm is preferred for this purpose.

Cellulose ethers particularly suitable for obtaining the emulsions of the present invention are water-soluble cellulose ethers described in Kirk-Othmer, "Encyclopedia of Chemical Technology", second edition, Vol. 4, page 616; examples of these products are carboxymethyl celluloses, carboxymethyl hydroxyethyl celluloses, hydroxyethyl celluloses, methyl celluloses, methylhydroxypropyl celluloses, ethylhydroxyethyl celluloses, methylethyl celluloses, methylhydroxyethyl celluloses, etc.

Particularly preferred cellulose ethers are carboxymethyl celluloses and carboxymethyl hydroxyethyl celluloses.

The most preferred ethers are carboxymethyl celluloses having a substitution degree higher than 0.5, generally 0.8, which provide a viscosity of the 2% aqueous solution, measured by means of a Hoeppler viscosimeter at 20 °C, of from 10 to 45000 mPa•s and a pH not higher than 8, generally of from 6 to 8.

The surface-active agent of general formula (III) can be added to the polyisocyanate either before emulsifying it or directly to the water already containing the cellulose ether.

In both cases the surface-active agent is used in amounts of from 0.5 to 2% by weight, based on the final emulsion.

The polyisocyanate may be added to the aqueous solution under agitation at room temperature and the system may then be kept at an agitation speed that is not too high, for instance, 1000 to 2000 rpm, for short periods of time, generally of from 30 to 60 seconds.

The polyisocyanate is usually added to the solution in quantities of at least 5% by weight, calculated on the total weight of the final emulsion, preferably in amounts of from 5 to 60% by weight.

The emulsions thus obtained show a high chemical and physical stability, in the most favourable cases for as long as 15 hours, which allows the use of these emulsions as binding agents for cellulosic materials on an industrial scale, without any risk of a product loss and/or damage of the apparatus, even in the case of interruptions due to unforeseen events.

Another advantage of the present invention is the possibility of making stable emulsions by using very limited amounts of stabilizers and without having to resort to expensive and complicated processes.

The chemical stability of the emulsion may be measured by determining the residual -NCO groups according to ASTM D 2572-80, whereas the physical stability may be monitored either by viscosity measurements by means of a Ford cup (diameter 4 mm, ASTM D 1200-82) or by determining the absence of clear floatings or settlings.

Also an object of the present invention are aqueous polyisocyanate emulsions which are obtainable by the process just described.

The aqueous emulsions of the present invention can be advantageously used as binding agents for cellulosic materials, preferably lignocellulosic materials such as wood, bark, bagasse, straw, bamboo, rice chaff etc., in the form of granulated products, chips, fibres or flours and, more particularly, for the

manufacture of particle boards or other composites such as plywoods, fibre boards, block boards, MDF boards, etc., without any problem as regards the industrial worker and the conservation of the emulsions. Generally, the emulsions are added to the (ligno)cellulosic material in a quantity of from 2 to 50% by weight, based on the cellulosic material, preferably of from 5 to 20% by weight.

Some illustrative but not limitative examples of embodiments of the present invention are given below.

EXAMPLE 1

(Preparation of surface-active urethane agents)

1a. 380 g of a polyoxyethylene glycol having an average molecular weight of about 400, 138 g of phenylisocyanate and 0.5 g of benzoyl chloride are introduced into a 1 l glass reactor equipped with thermometer, stirrer, cooler, nitrogen inlet and thermoregulation system. The mixture is heated to 80 °C and is allowed to react, under a nitrogen stream, until the -NCO groups have disappeared.
The reaction time is 4 to 5 hours.
Then the reaction product is allowed to cool to room temperature and is discharged.
1b. The operation is carried out as in 1a, but 300 g of a polyoxyethylene glycol having an average molecular weight of about 400, 180 g of phenylisocyanate and 0.5 g of benzoyl chloride are used.
The reaction time is 4 to 5 hours.
1c. The operation is carried out as in 1a, but 300 g of oxyethylated butanol having an average molecular weight of about 500, 83 g of phenylisocyanate and 0.3 g of benzoyl chloride are used.
The reaction time is 3 to 4 hours.
1d. The operation is carried out as in 1a, but 400 g of oxyethylated butanol having an average molecular weight of about 800, 59.1 g of phenylisocyanate and 0.3 g of benzoyl chloride are used.
The reaction time is about 7 hours.
1e. The operation is carried out as in 1a, but 500 g of polyoxyethylene glycol having an average molecular weight of about 1000, 92.6 g of phenylisocyanate and 0.5 g of benzoyl chloride are used.
The reaction time is 3 to 4 hours.
1f. The operation is carried out as in 1a, but 250 g of polyoxyethylene glycol having an average molecular weight of about 300, 200 g of phenylisocyanate and 0.4 g of benzoyl chloride are used.
The reaction time is 8 to 9 hours.

EXAMPLE 2

(Preparation of polyisocyanate emulsions)

2a. 590 g of a 1.6% aqueous carboxymethylcellulose (CMC) solution (substitution degree 0.82, Hoeppler viscosity of the 2% aqueous solution, measured at 20 °C, 100 mPa•s and pH 6.4) are introduced into a 2 l beaker and, thereafter, 10 g of surface-active urethane agent of example 1a and 400 g of polymethylene polyphenyl polyisocyanate (Tedimon(R) 31, Montedipe) are added under agitation (1800 rpm).
2b. The operation is carried out as in 2a, but the surface-active urethane agent of example 1b is used. The emulsion obtained shows the characteristics reported in table 1.
2c. The operation is carried out as in 2a, but the surface-active urethane agent of example 1c is used. The emulsion obtained shows the characteristics reported in table 1.
2d. The operation is carried out as in 2a, but the surface-active urethane agent of example 1d is used. The emulsion obtained shows the characteristics reported in table 1.
2e. The operation is carried out as in 2a, but the surface-active urethane agent of example 1e is used. The emulsion obtained shows the characteristics reported in table 1.
2f. The operation is carried out as in 2a, but 410 g of a mixture of Tedimon(R) 31 (400 g) and surface-active urethane agent of example 1f (10 g) are added to 590 g of the 1.6% aqueous CMC solution.

For the preparation of this mixture 400 g of Tedimon(R) 31 are introduced in a 1 l reactor and heated to 80 °C; 10 g of surface-active urethane agent are added and the resulting mixture is allowed to intimately mix at 80 °C for 10 minutes under a nitrogen stream.

The mixture obtained is cooled to room temperature, discharged and used for the preparation of the polyisocyanate emulsion.

The polyisocyanate emulsion shows the characteristics reported in table 1.

EXAMPLE 3

(Evaluations in the manufacturing of particle boards)

3a. Manufacturing of V20 particle boards (according to DIN 68763):

(i) 3000 g of wood chips of the type industrially used for the outer layers of particle boards and containing 5 to 6% of water are charged to a resin-applying machine; thereafter 400 g of the polyisocyanate emulsion of example 2a, diluted to 30% of dry matter, are sprayed onto the chips through suitable nozzles.

(ii) The above operation is repeated, using 3000 g of wood chips of the type industrially used for the inner layers of particle boards and containing 5 to 6% of water, spraying 200 g of the polyisocyanate emulsion of example 2a, diluted to 30% of dry matter, onto the chips.

On a 550 x 550 mm caul plate, which performs the function of supporting the mat, a layer of 700 g of resinated chips, obtained under (i), then a layer of 1400 g of resinated chips, obtained under (ii), and again a layer of 700 g of resinated chips (i) are placed. The mat is then charged to a press, in which it is subjected to a temperature of 175 ° C ± 5 ° C for 4 minutes.

A particle board is obtained which shows the characteristics reported in table 2.

3b. Manufacturing of V100 particle boards (according to DIN 68763):

The operation is carried out as in 3a, but the non-diluted polyisocyanate emulsion is used in quantities of 600 g of emulsion for 3000 g of chips (outer layers) and 450 g of emulsion for 3000 g of chips (inner layer of the particle board).

A particle board is obtained which shows the characteristics reported in table 2.

TABLE 1

Characteristics of the polyisocyanate emulsions

| Time | Example 2a | | Example 2b | | Example 2c | | Example 2d | | Example 2e | | Example 2f | |
|---|---|---|---|---|---|---|---|---|---|---|---|---|
| (hours) | Viscosity (seconds) | NCO content on starting value (%) | Viscosity (seconds) | NCO content on starting value (%) | Viscosity (seconds) | NCO content on starting value (%) | Viscosity (seconds) | NCO content on starting value (%) | Viscosity (seconds) | NCO content on starting value (%) | Viscosity (seconds) | NCO content on starting value (%) |
| 0 | 47 | 100 | 42 | 100 | 47 | 100 | 42 | 100 | 43 | 100 | 43 | 100 |
| 1 | 60 | | 50 | | 66 | | 56 | | 59 | | 49 | 97,6 |
| 2 | 64 | | 57 | | 70 | | 58 | | 61 | | 51 | |
| 3 | 64 | | 57 | | 69 | 95,9 | 58 | | 63 | | 52 | |
| 4 | 64 | | 58 | 95,7 | 69 | | 59 | | 65 | 96,3 | 52 | 90,4 |
| 5 | 63 | 94,8 | 56 | | 69 | 94,9 | 61 | | 67 | | 52 | |
| 6 | 63 | | 56 | | 69 | | 63 | | 69 | | 51 | |
| 7 | 66 | 94,2 | 59 | | 69 | | 66 | | 73 | | 52 | |
| 8 | 68 | " | 62 | 91,7 | 73 | 90,1 | 66 | | 79 | | 52 | 87,2 |
| 9 | 69 | " | 62 | | 75 | | 72 | 93,3 | 83 | 93,4 | 52 | |
| 10 | 74 | 92,3 | 64 | 89,3 | 78 | | 78 | | | | 58 | 83,8 |
| 11 | 79 | | 68 | | 82 | 88,6 | 81 | | | | | |
| 12 | 81 | 90,3 | 69 | 84,8 | 86 | 87,5 | 96 | 90,0 | | | | |
| 13 | 86 | | 70 | | | | | | | | | |
| 14 | 91 | 88,8 | 74 | | | | | | | | | |
| 15 | 96 | 87,2 | 77 | 81,9 | | | | | | | | |

Remark: The viscosity is measured at 20°C by means of Ford cup, nozzle 4 mm, according to ASTM D 1200-82

TABLE 2

| CHARACTERISTICS OF THE PARTICLE BOARDS | | |
|---|---|---|
| Characteristics | V20 particle board (Example 3a) | VI100 particle board (Example 3b) |
| Specific gravity kg/$m^3$ | 680 | 690 |
| Bending strength N/$mm^2$ | 17.0 | 18.1 |
| Tensile strength N/$mm^2$ | 0.57 | 0.32 |
| Swelling % | 15.0 | 9.2 |
| Moisture content % | 7.1 | 7.2 |
| Thickness mm | 16 | 16 |

## Claims

**1.** Process for the preparation of aqueous polyisocyanate emulsions, comprising the mixing of water and

(i) a cellulose ether in an amount of from 0.1 to 10% by weight, based on the total weight of water and cellulose ether;

(ii) a surface-active agent, derived from phenylisocyanate, of general formula (III):

$$X-(C_mH_{2m}O)_n-CONHY \qquad (III)$$

wherein

m is an integer of from 1 to 4;

n is an integer of from 5 to 40;

X represents hydroxy, a $C_1$-$C_4$ alkoxy or -CONHY group;

Y represents a phenylisocyanate residue;

in an amount of from 0.5 to 2% by weight of the final emulsion; and

(iii) at least one polyisocyanate selected from

(a) diisocyanates of general formula (I):

OCN-R-NCO (I)

wherein R represents an alkyl, cycloalkyl, aryl or alkylaryl radical having from 1 to 25 carbon atoms; and

(b) mixtures of polymethylene polyphenylisocyanates of general formula (II):

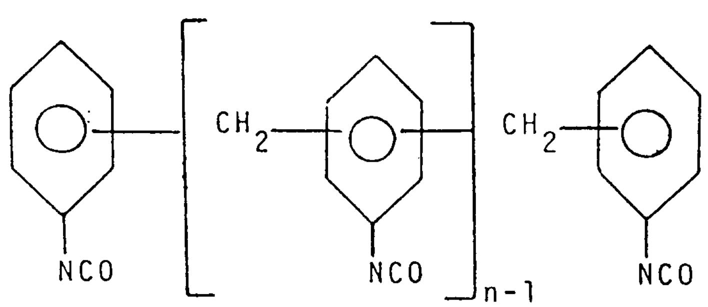

(II)

wherein n represents an integer higher than or equal to 1.

**2.** Process according to claim 1, wherein the surface-active agent of general formula (III) is selected from one or more of those obtained by the reaction of phenylisocyanate with polyoxyethylene glycols having an average molecular weight of from 200 to 1500, polyoxypropylene-ethylene glycols having an average molecular weight of from 200 to 1500, oxyethylated butanol having an average molecular weight of from 400 to 1000, oxyethylated methanol having an average molecular weight of from 500 to

2000, butanol oxyethylene-oxypropylene alcohol having an average molecular weight of from 500 to 1000, and methanol oxyethylene-oxypropylene alcohol having an average molecular weight of from 500 to 1000.

3. Process according to any one of claims 1 and 2, wherein the cellulose ether is dissolved in water, at room temperature, in an amount of from 0.5 to 5% by weight, based on the total weight of water and cellulose ether.

4. Process according to any one of the preceding claims, wherein the surface-active agent of general formula (III) is added to the polyisocyanate before emulsifying it, or is added directly to the water already containing the cellulose ether.

5. Process according to any one of the preceding claims, wherein the polyisocyanate is added to the aqueous solution while keeping the solution stirred.

6. Process according to any one of the preceding claims, wherein the polyisocyanate is added in amounts of at least 5% by weight, based on the final emulsion.

7. Aqueous emulsions, obtainable by the process of any one of claims 1 to 6.

8. Use of the emulsions of claim 7 as binding agents for cellulosic materials in the form of granulated products, chips, fibres or flours.

9. Use according to claim 8, wherein said cellulosic materials are ligno-cellulosic materials.

10. Use according to any one of claims 8 and 9, wherein the emulsions are used in an amount of from 2 to 50% by weight, based on the cellulosic material.

**Patentansprüche**

1. Verfahren zur Herstellung von wäßrigen Polyisocyanat-Emulsionen, umfassend das Mischen von Wasser und

   (i) einem Celluloseether in einer Menge von 0,1 bis 10 Gewichtsprozent, bezogen auf das Gesamtgewicht von Wasser und Celluloseether;

   (ii) einem Tensid, abgeleitet von Phenylisocyanat, der allgemeinen Formel (III):

$$X-(C_mH_{2m}O)_n-CONHY \qquad (III)$$

   worin
   m eine ganze Zahl von 1 bis 4 ist;
   n eine ganze Zahl von 5 bis 40 ist;
   X für Hydroxy, eine $C_1$-$C_4$-Alkoxy oder -CONHY-Gruppe steht;
   Y einen Phenylisocyanatrest darstellt;
   in einer Menge von 0,5 bis 2 Gewichtsprozent der endgültigen Emulsion; und

   (iii)mindestens einem Polyisocyanat, das ausgewählt ist aus

   (a) Diisocyanaten der allgemeinen Formel (I):

   OCN-R-NCO (I)

   worin R für einen Alkyl-, Cycloalkyl-, Aryl- oder Alkylaryl-Rest mit 1 bis 25 Kohlenstoffatomen steht; und

(b) Mischungen von Polymethylenpolyphenylisocyanaten der allgemeinen Formel (II)

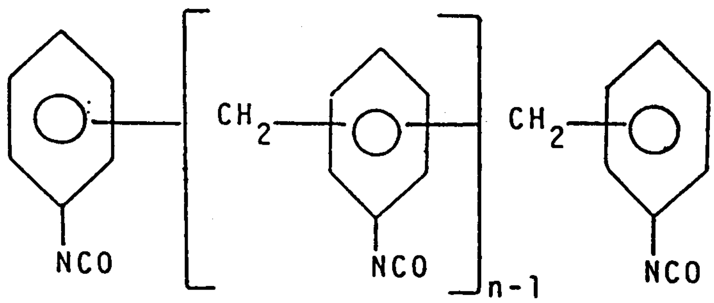

(II)

worin n eine ganze Zahl, die höher oder gleich 1 ist, darstellt.

2. Verfahren nach Anspruch 1, in welchem das Tensid der allgemeinen Formel (III) ausgewählt ist aus einem oder mehreren derjenigen, die erhalten werden durch die Reaktion von Phenylisocyanat mit Polyoxyethylenglycolen mit einem durchschnittlichen Molekulargewicht von 200 bis 1500, Polyoxypropylen-ethylen-glycolen mit einem durchschnittlichen Molekulargewicht von 200 bis 1500, oxyethyliertem Butanol mit einem durchschnittlichen Molekulargewicht von 400 bis 1000, oxyethyliertem Methanol mit einem durchschnittlichen Molekulargewicht von 500 bis 2000, Butanol-oxyethylen-oxypropylen-alkohol mit einem durchschnittlichen Molekulargewicht von 500 bis 1000 und Methanol-oxyethylen-oxypropylen-alkohol mit einem durchschnittlichen Molekulargewicht von 500 bis 1000.

3. Verfahren nach irgendeinem der Ansprüche 1 und 2, in welchem der Celluloseether bei Raumtemperatur in einer Menge von 0,5 bis 5 Gewichtsprozent, bezogen auf das Gesamtgewicht von Wasser und Celluloseether, in Wasser gelöst wird.

4. Verfahren nach irgendeinem der vorangehenden Ansprüche, in welchem das Tensid der allgemeinen Formel (III) dem Polyisocyanat vor dessen Emulgierung zugegeben wird oder direkt zu dem den Celluloseether bereits enthaltenden Wasser gegeben wird.

5. Verfahren nach irgendeinem der vorangehenden Ansprüche, in welchem das Polyisocyanat der wäßrigen Lösung zugegeben wird, während man die Lösung gerührt hält.

6. Verfahren nach irgendeinem der vorangehenden Ansprüche, in welchem das Polyisocyanat in Mengen von mindestens 5 Gewichtsprozent, bezogen auf die endgültige Emulsion, zugegeben wird.

7. Wäßrige Emulsionen, erhältlich durch das Verfahren nach irgendeinem der Ansprüche 1 bis 6.

8. Verwendung der Emulsionen von Anspruch 7 als Bindemittel für Cellulose-Materialien in Form von granulierten Produkten, Schnitzeln, Fasern oder Mehlen.

9. Verwendung nach Anspruch 8, worin die Cellulose-Materialien Lignocellulose-Materialien sind.

10. Verwendung nach irgendeinem der Ansprüche 8 und 9, worin die Emulsionen in einer Menge von 2 bis 50 Gewichtsprozent, bezogen auf das Cellulose-Material, verwendet werden.

**Revendications**

1. Procédé de préparation d'émulsions aqueuses de polyisocyanate, comprenant le mélange d'eau et
   (i) un éther de cellulose en une quantité de 0,1 à 10% en poids, par rapport au poids total de l'eau et de l'éther de cellulose;

(ii) un agent tensioactif obtenu à partir d'un phénylisocyanate représenté par la formule (III)

$$X—(C_mH_{2m}O)_n—CONHY— \qquad (III)$$

dans laquelle:

m  est un nombre entier de 1 à 4;
n  est un nombre entier de 5 à 40;
X  représente un groupe -OH, un groupe alkoxy en $C_1$-$C_4$ ou -CONHY et
Y  représente un résidu phénylisocyanate;

en une quantité de 0,5 à 2% par rapport au poids de l'émulsion finale, et

(iii) et au moins un polyisocyanate sélectionné parmi:

a) les diisocyanates de formule générale (I)

OCN-R-NCO  (I)

dans laquelle R représente un radical alkyle, cycloalkyle, aryle ou alkylaryle comportant de 1 à 25 atomes de carbone;

b) les mélanges de polyméthylène polyphénylisocyanates représentés par la formule générale (II) :

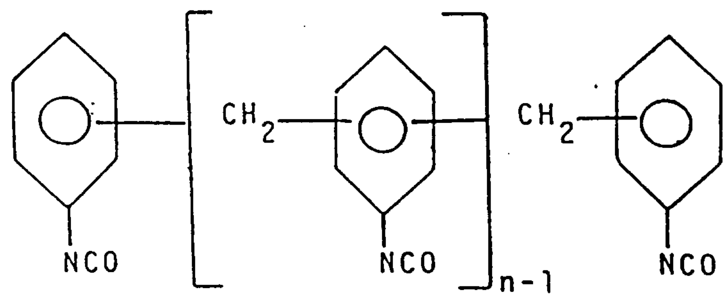
(II)

dans laquelle n représente un nombre entier supérieur ou égal à un.

**2.** Procédé selon la revendication 1, dans lequel l'agent tensioactif de formule générale (III) est sélectionné parmi un ou plusieurs des composés obtenus par la réaction de phénylisocyanate avec des polyoxyéthylèneglycols présentant un poids moléculaire moyen de 200 à 1 500, polyoxypropylèneéthylèneglycols présentant un poids moléculaire moyen de 200 à 1 500, butanol oxyéthylé présentant un poids moléculaire moyen de 400 à 1 000, méthanol oxyéthylé présentant un poids moléculaire moyen de 500 à 2 000, butanol oxyéthylène-alcool oxypropylénique présentant un poids moléculaire moyen de 500 à 1 000, et méthanol oxyéthylène-alcool oxypropylénique présentant un poids moléculaire moyen de 500 à 1 000.

**3.** Un procédé selon une des revendications 1 et 2, caractérisé en ce que l'éther de cellulose est dissous dans l'eau, à la température ambiante, en une quantité de 0,5 à 5% en poids, exprimé par rapport au poids total d'eau et d'éther de cellulose.

**4.** Un procédé selon une quelconque des revendications précédentes, caractérisé en ce que l'agent tensioactif de formule générale (III) est additionné au polyisocyanate avant l'étape d'émulsification de ce dernier ou est ajouté directement à l'eau contenant déjà l'éther de cellulose.

**5.** Procédé selon une quelconque des revendications précédentes, caractérisé en ce que le polyisocyanate est ajouté à la solution aqueuse alors que cette dernière est maintenue sous agitation.

**6.** Procédé selon une quelconque des revendications précédentes, caractérisé en ce que le polyisocyanate est ajouté en une proportion d'au moins 5 % en poids par rapport au poids de l'émulsion finale.

**7.** Emulsions aqueuses, qui peuvent être obtenues Par le procédé selon une quelconque des revendications 1 à 6.

**8.** Utilisation des émulsions selon la revendication 7 en tant que liants pour les matériaux cellulosiques sous forme de produits granulés, de copeaux, de fibres ou de farine.

**9.** Utilisation selon la revendication 8, caractérisée en ce que lesdits matériaux cellulosiques sont des matériaux ligno-cellulosiques.

**10.** Utilisation selon les revendications 8 et 9 caractérisée en ce que les émulsions sont utilisées en une quantité de 2 à 50% en poids par rapport à un matériaux cellulosique.